# EUROPÄISCHE PATENTSCHRIFT

(11) **EP 2 780 066 B1**
(45) Veröffentlichungstag und Bekanntmachung des Hinweises auf die Patenterteilung: **28.12.2016**
(21) Anmeldenummer: 12770083.9
(22) Anmeldetag: 27.09.2012
(51) Int. Cl.: A61M 16/08, A61M 16/10

(54) **SCHLAUCH FÜR EIN BEATMUNGSSYSTEM**
TUBE FOR A RESPIRATOR SYSTEM
TUYAU POUR SYSTÈME DE RESPIRATION

(30) Priorität: 17.11.2011 DE 102011055439
(43) Veröffentlichungstag der Anmeldung: 24.09.2014
(73) Patentinhaber: Hamilton Bonaduz AG, 7402 Bonaduz (CH)
(72) Erfinder: BÜCHI, Rudolf, 7000 Chur (CH); MAEDER, Marc, 7208 Malans (CH); ZOLKOS, Axel, 8864 Reichenburg (CH)
(74) Vertreter: Caspary, Karsten
(86) Internationale Anmeldenummer: PCT/EP2012/069149
(87) Internationale Veröffentlichungsnummer: WO 2013/072119

(56) Entgegenhaltungen:
- US-A- 5 392 770
- US-A1- 2007 079 982

## Beschreibung

Die vorliegende Erfindung betrifft einen Schlauch für ein Beatmungssystem zum Beatmen von Patienten mit Atemgas, insbesondere für ein Beatmungssystem für Neugeborene.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen in üblicher Weise mit destilliertem Wasser gefüllten Flüssigkeitsbehälter auf, durch den das Einatemgas geleitet und mit Feuchtigkeit angereichert wird.

Um eine Kondensation von Feuchtigkeit innerhalb des Beatmungsschlauchsystems zu verhindern, sind der Inspirationsschlauch und der Exspirations- oder Ausatemschlauch normalerweise mit einer elektrischen Schlauchheizung versehen, die in Betrieb das durchströmende Ein- oder Ausatemgas erwärmt. Dabei wird beispielsweise eine sich integral innerhalb des Inspirations- oder Exspirationsschlauchs erstreckende Heizdrahtschleife verwendet oder der Inspirations- oder Exspirationsschlauch ist jeweils mit einer Heizdrahtwendel umwickelt.

Die Regelung der Atemgastemperatur erfolgt im Allgemeinen mit Hilfe eines nahe am Patienten angeordneten Temperatursensors, der mittels einer elektrischen Messleitung mit einer Steuereinrichtung, die beispielsweise im Atemluftbefeuchter oder im Beatmungsgerät angeordnet ist, verbunden ist.

Bei der Beatmung von Neugeborenen oder Säuglingen in der Neonatologie besteht die Besonderheit darin, dass diese in Inkubatoren bzw. Brutkästen oder Wärmebetten liegen, deren Temperatur normalerweise oberhalb der Temperatur der klimatisierten Umgebung liegt, im Allgemeinen bei ca. 37°C. Deshalb muss z. B. der Bereich des Inspirationsschlauchs, der sich innerhalb des Inkubators oder Wärmebettes befindet, mit weniger bzw. gar keiner Heizleistung versorgt werden im Gegensatz zum außerhalb befindlichen Bereich, der zur Verhinderung der Kondensation weiter beheizt werden muss.

Bisherige Lösungsansätze für diese Problematik bestanden beispielsweise darin, den Inspirationsschlauch so auszubilden, dass der Temperatursensor je nach Anwendungsfall, d. h. entweder mit Inkubator oder bei einem Wärmebett mit Bestrahlung durch eine (Infrarot-) Wärmelampe, an zwei unterschiedlichen Position am Schlauch angebracht ist, und dass am Ende des Inspirationsschlauchs vor dem Y-Stück ein zusätzliches, unbeheiztes Erweiterungsschlauchstück eingesetzt wird.

Ein weiterer Ansatz ist in der DE 10 2007 003 455 A1 offenbart, bei der in den Inspirations- und Exspirationsschlauch ein Adapter eingefügt ist, der die gesamte Heizstrecke in zwei Teilheizstrecken innerhalb und außerhalb des Inkubators unterteilt, deren Beheizung getrennt voneinander regelbar ist.

Die DE 20 2006 007 397 U1 beschreibt einen Beatmungsschlauch mit unterschiedlichen Heizzonen, um den unterschiedlichen Klimazonen bei Säuglingsinkubatoren Rechnung zu tragen.

Die US 2007/0079982 A1 beschreibt einen Schlauch für ein Beatmungsgerät mit den Merkmalen gemäß des Oberbegriffs des Anspruchs 1.

Derartige Lösungsmodelle sind kompliziert zu bedienen, aufwändig und erzeugen damit hohe Kosten. Durch die Tatsache, dass bei Beatmungssystemen das Schlauchsystem aus Hygienegründen häufig als medizinischer Einmal- bzw. Wegwerfartikel ausgebildet ist, sind derartige Ansätze diesbezüglich nicht zielführend.

Es ist daher die Aufgabe der vorliegenden Erfindung, einen Schlauch für ein Beatmungssystem bereit zu stellen, der kostengünstig herstellbar ist, flexible und einfache Handhabung und Einsatzmöglichkeiten bietet und in allen Anwendungsfällen für eine zuverlässige Beheizung und damit eine ordnungsgemäße Beatmung ermöglicht.

Diese Aufgabe wird durch die Merkmale des Anspruchs 1 gelöst. Vorteilhafte Ausgestaltungen und Ausführungsformen sind Gegenstand der Unteransprüche.

Erfindungsgemäß wird ein Schlauch für ein Beatmungssystem für Neugeborene bereitgestellt mit einem ersten Abschnitt, der einen Heizdraht, eine elektrische Leitung und ein erstes und ein zweites Anschlussstück aufweist, und einem zweiten Abschnitt, der zumindest abschnittsweise einen Heizdraht und zumindest abschnittsweise eine elektrische Leitung sowie ein erstes und ein zweites Anschlussstück aufweist, wobei der zweite Abschnitt einen Temperatursensor aufweist, wobei die elektrische und pneumatische Verbindung des ersten Abschnitts mit dem zweiten Abschnitt über die Verbindung des zweiten Anschlussstücks des ersten Abschnitts in einer ersten Konfiguration möglich ist wobei das zweite Anschlussstück des ersten Abschnitts mit dem ersten Anschlussstück des zweiten Abschnitts verbunden ist, so dass der zweite Abschnitt beheizbar ist. Alternativ ist in einer zweiten Konfiguration die elektrische und pneumatische Verbindung des ersten Abschnitts mit dem zweiten Abschnitt über die Verbindung des zweiten Anschlussstücks des ersten Abschnitts möglich, wobei das zweite Anschlussstück des ersten Abschnitts mit dem zweiten Anschlussstück des zweiten Abschnitts verbunden ist und ein Verbindungsstück am oder im Anschlussstück den Heizdraht kurzschließt, so dass der zweite Abschnitt im Wesentlichen nicht beheizbar ist. Damit ist der Schlauch als Kombination des ersten Abschnitts mit dem zweiten Abschnitt in mehreren Konfigurationen flexibel verwendbar. Es kann der gesamte Schlauch oder auch nur ein Teil davon beheizt werden, ohne die (äußeren) Einstellungen der Heizung zu verändern, d. h. alleine das Umdrehen des zweiten Abschnitts (Vertauschen der Anschlussstücke) bewirkt die veränderte Heizkonfiguration.

Vorzugsweise ist der Schlauch als Inspirationsschlauch ausgebildet, wobei der erste Abschnitt mit einem Atemluftbefeuchter und der zweite Abschnitt mit einem Y-Stück nahe am Patienten verbindbar sind. Dies stellt die Standardanwendung des erfindungsgemäßen Schlauches dar. Alternativ ist denkbar, dass ein erfindungsgemäßer Schlauch auch als Exspirationsschlauch verwendet wird.

Bevorzugt ist der Heizdraht des ersten und/oder des zweiten Abschnitts als hochohmige Zweidrahtleitung ausgebildet. Damit kann die Zweidrahtleitung als Widerstandsheizung genutzt werden, die beispielsweise spiralförmig in die Schlauchwand integriert ist. Alternativ sind auch mehr als zwei hochohmige Heizdrähte möglich. Auch sind andere Konfigurationen außer der Spiralform denkbar, z.B. eine Längsanordnung oder zickzack-förmig.

Vorteilhafterweise ist die elektrische Leitung des ersten und/oder des zweiten Abschnitts als niederohmige zweidrahtige Signal- oder Messleitung ausgebildet und beispielsweise in die Schlauchwand integriert. Damit sind auf einfache Weise der Temperatursensor oder andere Sensoren anschließbar. Zusätzlich können auch noch weitere Signal- oder Messleitungen, beispielsweise zur Datenübertragung, vorhanden sein. Die Signal- und Messleitungen können beispielsweise parallel zu den Heizleitungen angeordnet sein. Es ist alternativ auch eine (thermische) Entkopplung der jeweiligen Leitungen möglich.

Besonders bevorzugt ist der Temperatursensor als Thermoelement ausgebildet und in das zweite Anschlussstück des zweiten Abschnitts integriert, wobei das Thermoelement geeignet ist, die niederohmige zweidrahtige Signal- oder Messleitung zu überbrücken und wobei eine Verbindung im zweiten Anschlussstück des zweiten Abschnitts geeignet ist, die hochohmige Heizzweidrahtleitung zu überbrücken. Damit ist eine besonders einfache Ausbildung des zweiten Abschnitts gegeben, der einfach umgedreht werden kann, damit der Schlauch die andere Konfiguration annehmen kann. Mit der Integration des Thermoelements in das Anschlussstück wird der Ort für die Temperaturmessung optimal einsetzbar. Die Überbrückung der elektrischen Verbindung im zweiten Anschlussstück des zweiten Abschnitts schließt die Heizleitung kurz und unterbindet damit wirksam die Beheizung des zweiten Abschnitts in der zweiten Konfiguration.

Mit weiterem Vorteil sind die elektrischen Kontakte der Anschlussstücke derart ausgebildet, dass ein unbeabsichtigtes Berühren der elektrischen Kontakte von außen nicht möglich ist. Dadurch wird verhindert, dass die Kontakte kurzgeschlossen werden und eine Beheizung unbeabsichtigt auftritt oder die Signal- oder Messleitung nicht mehr funktionsfähig ist. Auch wird dadurch eine Beschädigung der elektrischen Kontakte weitgehend verhindert.

Bevorzugt ist der erste Abschnitt länger als der zweite Abschnitt ausgebildet.

Erfindungsgemäß ist ebenfalls ein Beatmungsschlauchsystem mit einem Inspirationsschlauch, einem Exspirationsschlauch und einem wie oben definierten Schlauch. Ein derartiges Beatmungsschlauchsystem ist Teil eines Beatmungssystems und verbindet üblicherweise ein Beatmungsgerät, einen Atemluftbefeuchter und ein Y-Stück derart, dass ein Patient beatmet werden kann.

Mit besonderem Vorteil ist das Beatmungsschlauchsystem als medizinischer Einmal- bzw. Wegwerfartikel ausgebildet. Damit wird den Anforderungen an die Hygiene im Krankenhaus Rechnung getragen. Üblicherweise wird das gesamte Beatmungsschlauchsystem, gegebenenfalls auch mit einem Flüssigkeitsbehälter des Atemluftbefeuchters, in eine Kunststofffolie eingeschweißt geliefert, eingesetzt und nach Verwendung bei einem Patienten wieder entfernt und ausgetauscht. Durch die Flexibilität des erfindungsgemäßen Schlauchs ist das Beatmungsschlauchsystem für unterschiedliche Anwendungen in der Neonatologie oder auch bei der Beatmung von erwachsenen Patienten geeignet.

Die Erfindung soll nachfolgend anhand von Ausführungsbeispielen unter Bezugnahme auf die beigefügten Figuren erläutert werden, in denen:
- Fig. 1: eine schematische Darstellung einer bevorzugten Ausführungsform eines erfindungsgemäßen Schlauchs in einem Beatmungssystem zeigt; und
- Fig. 2: eine schematische Darstellung der bevorzugten Ausführungsform des Schlauchs gemäß der vorliegenden Erfindung in einer ersten Konfiguration zeigt,
- Fig. 3: eine schematische Darstellung der bevorzugten Ausführungsform des Schlauchs gemäß der vorliegenden Erfindung in einer zweiten Konfiguration zeigt, und
- Fig. 4: eine schematische Darstellung der Anschlussstücke in einer Ausführungsform des Schlauchs gemäß der vorliegenden Erfindung zeigt.

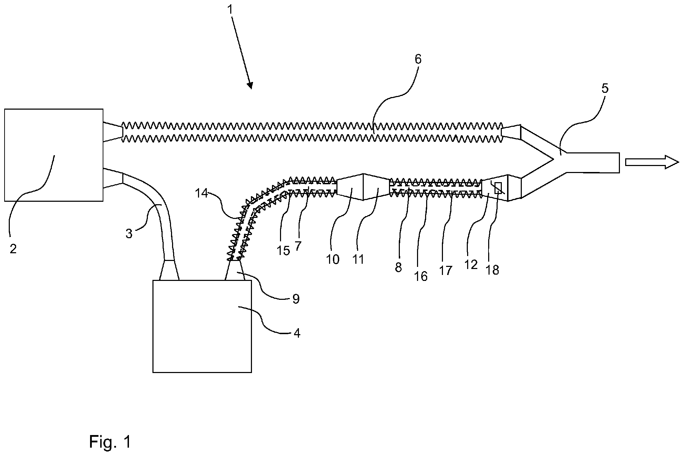

Fig. 1 zeigt in schematischer Darstellung eine bevorzugte Ausführungsform eines erfindungsgemäßen Schlauchs, der in einem Beatmungssystem 1 eingesetzt ist. Ein erster Inspirationsschlauch 3 verbindet ein Beatmungsgerät 2 mit einem Atemluftbefeuchter 4. Der Atemluftbefeuchter 4 ist über den erfindungsgemäßen Schlauch mit einem Y-Stück 5 verbunden. Das einfach ausgebildete Ende des Y-Stücks 5 zeigt mit dem dargestellten Pfeil in Richtung des zu beatmenden Patienten. Schließlich ist ein Exspirationsschlauch 6 zwischen dem Beatmungsgerät 2 und dem verbleibenden Ende des Y-Stücks 5 angeordnet.

Trockenes Atemgas wird beispielsweise über ein (nicht dargestelltes) Gebläse im Beatmungsgerät 2 erzeugt und verlässt dieses über den ersten Inspirationsschlauch 3 in Richtung des Atemluftbefeuchters 4. Dort wird das Atemgas in bekannter Art und Weise in einen (in Fig. 1 nicht dargestellten) Flüssigkeitsbehälter geleitet, wo es durch die aufgeheizte Flüssigkeit wie beispielsweise destilliertes Wasser erwärmt und befeuchtet wird. Über den Schlauch, der aus der Kombination eines ersten Abschnitts 7 mit einem zweiten Abschnitt 8 gebildet ist, verlässt das erwärmte und befeuchtete Atemgas den Atemluftbefeuchter 4 und wird dem Patienten über das Y-Stück 5 zugeführt. Entsprechend einem über das Beatmungsgerät 2 gesteuerten Atemzyklus verlässt die verbrauchte Atemluft den Patienten wieder und tritt am Y-Stück 5 in den Exspirationsschlauch 6 ein und wird wieder dem Beatmungsgerät 2 zugeführt.

In der hier dargestellten ersten Konfiguration ist der erste Abschnitt 7 über ein erstes Anschlussstück 9 mit dem Atemluftbefeuchter 4 und über ein zweites Anschlussstück 10 mit einem ersten Anschlussstück 11 des zweiten Abschnitts 8 verbunden. Ein zweites Anschlussstück 12 des zweiten Abschnitts 8 verbindet diesen mit einem Anschlussstück 13 des Y-Stücks 5.

In die Schlauchwand des ersten Abschnitts 7 ist ein Heizdraht 14 integriert, der beispielsweise spiralförmig als zweidrahtige, hochohmige Widerstandsheizwendel ausgebildet ist. Die Stromversorgung des Heizdrahtes 14 erfolgt durch elektrische Anschlüsse in dem ersten Anschlussstück 9, das den ersten Abschnitt 7 mit dem Atemluftbefeuchter 4 verbindet. Weiterhin in den ersten Abschnitt 7 integriert ist eine elektrische Signalleitung 15, die ebenfalls zweidrahtig, d.h. mit zwei Leitern , ausgebildet und geeignet ist, ein elektrisches Signal vom Atemluftbefeuchter 4 zum zweiten Abschnitt 8 und/oder in umgekehrter Richtung zu übertragen.

Der zweite Abschnitt 8 umfasst einen Heizdraht 16, der beispielsweise spiralförmig als zweidrahtige, hochohmige Widerstandsheizwendel ausgebildet ist und der sich ausgehend vom ersten Anschlussstück 11 im Wesentlichen bis hin zum zweiten Anschlussstück 12 erstreckt. In den zweiten Abschnitt 8 integriert ist auch eine elektrische Signalleitung 17, die ein im zweiten Anschlussstück 12 des zweiten Abschnitts 8 angeordnetes Thermoelement 18 elektrisch mit dem ersten Anschlussstück 11 verbindet. Das Messsignal des Thermoelements 18 wird vom Anschlussstück 11 über die elektrische Signalleitung 17 zum Anschlussstück 11 und von dort über das Anschlussstück 10, die elektrische Signalleitung 15 und das Anschlussstück 9 zum (nicht dargestellten) Steuergerät des Atemluftbefeuchters 4 übertragen. Mit anderen Worten: die Anschlusstücke 10, 11 der ersten und zweiten Abschnitte 7, 8 sind so ausgebildet, dass die entsprechenden elektrischen Kontakte die dazu gehörigen elektrischen Heiz- und Signalleitungen 14, 15, 16 bzw. 17 miteinander verbinden, so dass im Wesentlichen ein zweidrahtiger, hochohmiger Heizdraht sowie eine zweidrahtige, niederohmige Mess- oder Signalleitung über beide Abschnitte 7, 8 hinweg gebildet ist. In dieser ersten Konfiguration ist der gesamte, durch die beiden Abschnitte 7, 8 gebildete Schlauch beheizbar, und die Temperatur wird im Wesentlichen an dem Ende des Schlauchs gemessen, das nahe am Y-Stück 5 liegt.

Bei der zweiten, hier nicht dargestellten Konfiguration ist der zweite Abschnitt 8 genau umgekehrt angeordnet, d.h. das zweite Anschlussstück 12 mit dem Thermoelement 18 ist direkt mit dem zweiten Anschlussstück 10 des ersten Abschnitts 7 verbunden, und das erste Anschlussstück 11 des zweiten Abschnitts 8 mit dem Y-Stück 5. Dabei sorgt eine kurze Überbrückungsleitung im zweiten Anschlussstück 12 dafür, dass der Heizdraht 14 des ersten Abschnitts 7 elektrisch nicht mehr mit dem Heizdraht 16 des zweiten Abschnitts 8 verbunden ist und somit keine Beheizung des zweiten Abschnitts 8 mehr erfolgt. In ähnlicher Weise verbindet eine elektrische Leitung innerhalb des zweiten Anschlussstücks 12 die beiden Elemente der elektrischen (Zweidraht-) Signalleitung 15 derart, dass die Messsignale des Thermoelements 18 übertragen werden können. In der zweiten Konfiguration ist demnach lediglich der erste Abschnitt 7 beheizbar und die Temperatur ist am Ort des zweiten Anschlussstücks 12 im Wesentlichen am Verbindungspunkt der ersten und zweiten Abschnitte 7 und 8 mittels des Thermoelements 18 messbar.

Auch der Exspirationsschlauch 6 kann eine Schlauchheizung in Form eines Heizdrahtes aufweisen, der ebenfalls als Heizwendel spiralförmig ausgebildet sein kann. Der Grund für die Beheizung des Exspirationsschlauches 6 ist, dass vermieden werden soll, dass zurückströmendes Atemgas im Exspirationsschlauch 6 kondensiert und beispielsweise als verunreinigte Flüssigkeit über das Y-Stück 5 wieder zurück zum Patienten fließt. Die Schlauchheizung des Exspirationsschlauches 6 kann durchgängig oder abschnittsweise ausgebildet sein. Gemäß der vorliegenden Erfindung ist es möglich, dass der erfindungsgemäße zweiteilige Schlauch als Exspirationsschlauch eingesetzt wird. Dies erhöht die Flexibilität der Anwendung des Beatmungssystems noch weiter.

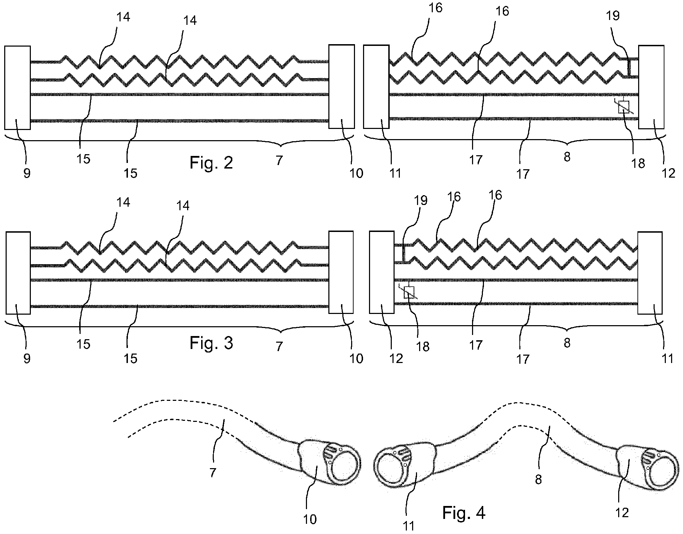

Fig. 2 zeigt eine schematische Darstellung des erfindungsgemäßen Schlauchs in der ersten Konfiguration, d.h. ähnlich zu der in Fig. 1 dargestellten Konfiguration. Der erste Abschnitt 7 weist die zweidrahtige, hochohmige Heizdrahtleitung 14 und die niederohmige elektrische Mess- oder Signalleitung 15 zwischen dem ersten Anschlussstück 9 und dem zweiten Anschlussstück 10 auf. Der zweite Abschnitt 8, der etwas kürzer als der erste Abschnitt 7 dargestellt ist, weist die zweidrahtige, hochohmige Heizdrahtleitung 16 und die niederohmige elektrische Mess- oder Signalleitung 17 zwischen dem ersten Anschlussstück 11 und dem zweiten Anschlussstück 12 auf. Dabei verbindet der als Thermoelement ausgebildete Temperatursensor 18 im Wesentlichen am oder im Anschlussstück 12 die beiden Leitungselemente der elektrischen Mess- oder Signalleitung 17, und die schematisch als Verbindungsstück 19 dargestellte Leitung verbindet die beiden Leitungselemente der Heizdrahtleitung 16 ebenfalls im Wesentlichen am oder im Anschlussstück 12.

Die Verbindung der Anschlussstücke 10 und 11 führt den elektrischen Kontakt der Elemente der Heizdrahtleitung 14 mit den entsprechenden Elementen der Heizdrahtleitung 16 und der Elemente der elektrischen Mess- oder Signalleitung 15 mit den entsprechenden Elementen der elektrischen Mess- oder Signalleitung 17 herbei.

Fig. 3 zeigt eine schematische Darstellung des erfindungsgemäßen Schlauchs in der zweiten Konfiguration, wobei der zweite Abschnitt 8 im Vergleich zur ersten Konfiguration umgedreht ist, d.h. die Anschlussstücke sind vertauscht. Der erste Abschnitt 7 ist unverändert wie in Fig. 1 dargestellt. Der zweite Abschnitt 8 ist nun mittels des zweiten Anschlussstücks 12 mit dem zweiten Anschlussstück 10 des ersten Abschnitts 7 verbunden.

Nunmehr misst der als Thermoelement ausgebildete Temperatursensor 18 im Wesentlichen am oder im Anschlussstück 12 direkt die Temperatur, wobei sein Messsignal über die elektrische Verbindung der beiden Anschlussstücke 10 und 11 und die elektrische Mess- oder Signalleitung 15 des ersten Abschnitts 7 zum (nicht dargestellten) Steuergerät übertragen wird, und die schematisch als Verbindungsstück 19 dargestellte Leitung schließt die beiden Leitungselemente der Heizdrahtleitung 16 im Wesentlichen am oder im Anschlussstück 12 derart kurz, so dass keine Beheizung des zweiten Abschnitts 8 erfolgen kann.

Die Anschlussstücke 10, 11 und 12 sind derart konfiguriert, dass das Umdrehen des zweiten Abschnitts 8 ohne Auswirkung auf die wirksame elektrische Verbindung der entsprechenden Kontakte ist. Dies kann durch geeignete bekannte Kontaktausführungen bewerkstelligt werden. Insbesondere ist dabei darauf zu achten, dass die Verbindung zwischen den Anschlussstücken einfach herzustellen und wieder zu lösen sein soll und gleichzeitig eine für die reibungslose Atemgasübertragung ausreichend gewährleistet sein muss. Es können beispielsweise Schraub-, Dreh-, Steck-, Feder- oder auch magnetische Verbindungsstrukturen zum Einsatz kommen.

Fig. 4 zeigt eine schematische Darstellung der Anschlussstücke in einer Ausführungsform des erfindungsgemäßen Schlauchs. Die elektrischen Kontakte sind in den Anschlussstücken 10, 11 und 12 in den Stirnflächen angeordnet. Man erkennt, dass die Anschlussstücke 11 und 12 des zweiten Abschnitts 8 im Wesentlichen symmetrisch aufgebaut sind, so dass beide mit dem Anschlussstück 10 des ersten Abschnitts 7 verbindbar sind.

Das Material für den Schlauch bzw. die Schläuche, d. h. für den Inspirationsschlauch 3, die ersten und zweiten Abschnitte 7 und 8 sowie den Exspirationsschlauch 6 ist aus einem geeigneten Kunststoffmaterial wie z. B. Polyethylen oder Polypropylen gebildet. Andere geeignete Materialien sind ebenfalls möglich. Die Schläuche werden in bekannter Technik extrudiert bzw. koextrudiert. Der Innendurchmesser der Schläuche beträgt bei Beatmungssystemen in der Neonatologie üblicher Weise etwa 12 mm oder 15 mm, es können allerdings auch größere Durchmesser wie z.B. 19 mm verwendet werden, beispielsweise bei Beatmungssystemen für Erwachsene. Die Anschlussstücke, die den Übergang zwischen den Schläuchen der entsprechenden Geräte bzw. dem Y-Stück 5 bilden, sind ebenfalls aus Kunststoffmaterial extrudiert. Da im medizinischen Bereich an die Materialien hohe Anforderungen gestellt werden, müssen die Materialen der ISO-Norm 5367-2000 genügen. Wie bereits erwähnt ist der erfindungsgemäße Schlauch als Teil eines Beatmungsschlauchsystems entweder als medizinischer Einmal-/Wegwerfartikel ausgebildet oder alternativ als wieder verwendbarer medizinischer Artikel, der mittels Waschen und Autoklavieren wieder in einen neu verwendbaren Zustand versetzt werden kann. Alle Bestandteile des Schlauchs müssen auch derart ausgebildet sein, dass sie beispielsweise keine Schadstoffe enthalten und einem kalten Desinfektionsmittel wie z.B. CIDEX, Sekusept, Korsolex usw. widerstehen.

Mit dem Gegenstand der vorliegenden Erfindung wurde ein Schlauch für ein Beatmungssystem für Neugeborene bereit gestellt, der kostengünstig herstellbar ist, flexible Einsatzmöglichkeiten bietet und in allen Anwendungsfällen für eine zuverlässige Beheizung und damit eine ordnungsgemäße Beatmung ermöglicht.

## Patentansprüche

1. Schlauch für ein Beatmungssystem (1) für Neugeborene mit
einem ersten Abschnitt (7), der einen Heizdraht (14), eine elektrische Leitung (15) und ein erstes (9) und ein zweites Anschlussstück (10) aufweist, und
einem zweiten Abschnitt (8), der zumindest abschnittsweise einen Heizdraht (16) und zumindest abschnittsweise eine elektrische Leitung (17) sowie ein erstes (11) und ein zweites Anschlussstück (12) aufweist,
wobei der zweite Abschnitt (8) einen Temperatursensor (18) aufweist,
wobei die elektrische und pneumatische Verbindung des ersten Abschnitts (7) mit dem zweiten Abschnitt (8) über die Verbindung des zweiten Anschlussstücks (10) des ersten Abschnitts (7) in einer ersten Konfiguration möglich ist, wobei das zweite Anschlussstück (10) des ersten Abschnitts (7) mit dem ersten Anschlussstück (11) des zweiten Abschnitts (8) verbunden ist, so dass der zweite Abschnitt (8) beheizbar ist;
**dadurch gekennzeichnet, dass**
die elektrische und pneumatische Verbindung des ersten Abschnitts (7) mit dem zweiten Abschnitt (8) über die Verbindung des zweiten Anschlussstücks (10) des ersten Abschnitts (7) alternativ in einer zweiten Konfiguration möglich ist, wobei das zweite Anschlussstück (10) des ersten Abschnitts (7) mit dem zweiten Anschlussstück (12) des zweiten Abschnitts (8) verbunden ist und ein Verbindungsstück (19) am oder im Anschlussstück (12) den Heizdraht (16) kurzschließt, so dass der zweite Abschnitt (8) im Wesentlichen nicht beheizbar ist.

2. Schlauch nach Anspruch 1, **dadurch gekennzeichnet, dass** er als Inspirationsschlauch ausgebildet ist, wobei der erste Abschnitt (7) mit einem Atemluftbefeuchter (4) und der zweite Abschnitt (8) mit einem Y-Stück (5) nahe am Patienten verbindbar ist.

3. Schlauch nach einem der vorhergehenden Ansprüche, **dadurch gekennzeichnet, dass** der Heizdraht (14, 16) des ersten (7) und/oder des zweiten Abschnitts (8) als hochohmige Zweidrahtleitung ausgebildet ist.

4. Schlauch nach einem der vorhergehenden Ansprüche, **dadurch gekennzeichnet, dass** die elektrische Leitung (15, 17) des ersten (7) und/oder des zweiten Abschnitts (8) als niederohmige zweidrahtige Signal- oder Messleitung ausgebildet ist.

5. Schlauch nach Anspruch 3 und 4, **dadurch gekennzeichnet, dass** der Temperatursensor (18) als Thermoelement ausgebildet und in das zweite Anschlussstück (12) des zweiten Abschnitts (8) integriert ist, wobei das Thermoelement geeignet ist, die niederohmige zweidrahtige Signal- oder Messleitung (17) zu überbrücken und wobei eine Verbindung im oder am zweiten Anschlussstück (12) des zweiten Abschnitts (8) geeignet ist, die hochohmige Heizzweidrahtleitung (16) zu überbrücken.

6. Schlauch nach einem der vorhergehenden Ansprüche, **dadurch gekennzeichnet, dass** die elektrischen Kontakte der Anschlussstücke (10, 11, 12) derart ausgebildet sind, dass ein unbeabsichtigtes Berühren der elektrischen Kontakte von außen nicht möglich ist.

7. Schlauch nach einem der vorhergehenden Ansprüche, **dadurch gekennzeichnet, dass** der erste Abschnitt (7) länger als der zweite Abschnitt (8) ausgebildet ist.

8. Beatmungsschlauchsystem mit einem Inspirationsschlauch (3), einem Exspirationsschlauch (6) und einem Schlauch nach einem der vorhergehenden Ansprüche.

9. Beatmungsschlauchsystem nach Anspruch 8, **dadurch gekennzeichnet, dass** es als medizinischer Einmal- bzw. Wegwerfartikel ausgebildet ist.

## Claims

1. Tube for a respirator system (1) for new-borns with
a first portion (7) which has a heating wire (14), an electric lead (15) and a first (9) and a second connector (10), and
a second portion (8) which has at least in some portions a heating wire (16) and at least in some portions an electric lead (17) as well as a first (11) and a second connector (12),
wherein the second portion (8) has a temperature sensor (18),
wherein the electrical and pneumatic connection of the first portion (7) to the second portion (8) via the connection of the second connector (10) of the first portion (7) is possible in a first configuration, wherein the second connector (10) of the first portion (7) is connected to the first connector (11) of the second portion (8) so that the second portion (8) can be heated;
**characterised in that** the electrical and pneumatic connection of the first portion (7) to the second portion (8) via the connection of the second connector (10) of the first portion (7) is possible alternatively in a second configuration, wherein the second connector (10) of the first portion (7) is connected to the second connector (12) of the second portion (8) and a connecting member (19) on or in the connector (12) short-circuits the heating wire (16) so that the second portion (8) essentially cannot be heated.

2. Tube according to claim 1, **characterised in that** it is configured as an inspiratory tube wherein the first portion (7) can be connected to a respiratory humidifier (4) and the second portion (8) can be connected to a Y-member (5) close to the patient.

3. Tube according to one of the preceding claims, **characterised in that** the heating wire (14, 16) of the first (7) and/or second portion (8) is configured as a high-impedance twin-wire lead.

4. Tube according to one of the preceding claims, **characterised in that** the electrical lead (15, 17) of the first (7) and/or second portion (8) is configured as a low-impedance twin-wire signal or measuring lead.

5. Tube according to claims 3 and 4 **characterised in that** the temperature sensor (18) is designed as a thermo element and is integrated into the second connector (12) of the second portion (8), wherein the thermo element is suitable for bridging the low-impedance twin-wire signal or measuring lead (17) and wherein a connection in or on the second connector (12) of the second portion (8) is suitable for bridging the high-impedance twin-wire heating lead (16).

6. Tube according to one of the preceding claims **characterised in that** the electrical contacts of the connectors (10, 11, 12) are configured in such a way that accidental contact of the electrical contacts from outside is not possible.

7. Tube according to one of the preceding claims, **characterised in that** the first portion (7) is configured longer than the second portion (8).

8. Respiratory tube system with an inspiratory tube (3), an expiratory tube (6) and a tube according to one of the preceding claims.

9. Respiratory tube system according to claim 8 **characterised in that** it is configured as a single-use or disposable medical article.

## Revendications

1. Tuyau pour système de respiration (1) pour nouveau-nés comportant
un premier segment (7) qui présente un fil chauffant (4), un câble électrique (15) et une première (9) et une deuxième pièces de raccordement (10), et
un deuxième segment (8) qui présente au moins par segment, un fil chauffant (16) et au moins par segment, un câble électrique (17) ainsi qu'une première (11) et une deuxième pièces de raccordement (12),
dans lequel le deuxième segment (8) présente un capteur de température (18),
dans lequel la liaison électrique et pneumatique du premier segment (7) avec le deuxième segment (8) est possible par la liaison de la deuxième pièce de raccordement (10) du premier segment (7) dans une première configuration, dans lequel la deuxième pièce de raccordement (10) du premier segment (7) est reliée à la première pièce de raccordement (11) du deuxième segment (8) de sorte que le deuxième segment (8) puisse être chauffé ;
**caractérisé en ce que**
la liaison électrique et pneumatique du premier segment (7) avec le deuxième segment (8) est possible par la liaison de la deuxième pièce de raccordement (10) du premier segment (7) en variante dans une deuxième configuration, dans lequel la deuxième pièce de raccordement (10) du premier segment (7) est reliée à la deuxième pièce de raccordement (12) du deuxième segment (8) et une pièce de liaison (19) sur ou dans la pièce de raccordement (12) court-circuite le fil chauffant (16) de sorte que le deuxième segment (8) ne puisse sensiblement pas être chauffé.

2. Tuyau selon la revendication 1, **caractérisé en ce qu'**il est formé comme un tube d'inspiration, dans lequel le premier segment (7) peut être relié à un humidificateur d'air de respiration (4) et le deuxième segment (8) peut être relié à une pièce en Y (5) à proximité du patient.

3. Tuyau selon l'une des revendications précédentes, **caractérisé en ce que** le fil chauffant (14, 16) du premier (7) et/ou du deuxième segment (8) est formé comme un câble bifilaire à valeur ohmique élevée.

4. Tuyau selon l'une des revendications précédentes, **caractérisé en ce que** le câble électrique (15, 17) du premier (7) et/ou du deuxième segment (8) est formé comme un câble bifilaire de signal ou de mesure.

5. Tuyau selon la revendication 3 ou 4, **caractérisé en ce que** le capteur de température (18) est formé comme un thermo-élément et est intégré dans la deuxième pièce de raccordement (12) du deuxième segment (8), dans lequel le thermo-élément est adapté pour ponter le câble bifilaire de signal ou de mesure (17) à faible valeur ohmique et dans lequel une liaison dans ou sur la deuxième pièce de raccordement (12) du deuxième segment (8) est adaptée pour ponter le câble bifilaire chauffant à valeur ohmique élevée.

6. Tuyau selon l'une des revendications précédentes, **caractérisé en ce que** les contacts électriques des pièces de raccordement (10, 11, 12) sont formés de telle sorte qu'un contact involontaire des contacts électrique de l'extérieur ne soit pas possible.

7. Tuyau selon l'une des revendications précédentes, **caractérisé en ce que** le premier segment (7) est plus long que le deuxième segment (8).

8. Système de tuyau de respiration comportant un tuyau d'inspiration (3), un tuyau d'expiration (6) et un tuyau selon l'une des revendications précédentes.

9. Système de tuyau de respiration selon la revendication 8, **caractérisé en ce qu'**il est formé comme un article médical à usage unique ou jetable.
